(19) Europäisches Patentamt
European Patent Office
Office européen des brevets

(11) Veröffentlichungsnummer: **0 158 598**
**B1**

(12) # EUROPÄISCHE PATENTSCHRIFT

(45) Veröffentlichungstag der Patentschrift: **04.01.89**

(51) Int. Cl.⁴: **C 07 D 211/46**, C 07 D 211/58, C 07 D 491/10, C 08 K 5/34 // (C07D491/10, 317:00, 221:00)

(21) Anmeldenummer: **85810148.8**

(22) Anmeldetag: **02.04.85**

(54) **Neue Piperidinverbindungen.**

(30) Priorität: **09.04.84 IT 2044984**

(43) Veröffentlichungstag der Anmeldung: **16.10.85 Patentblatt 85/42**

(45) Bekanntmachung des Hinweises auf die Patenterteilung: **04.01.89 Patentblatt 89/1**

(84) Benannte Vertragsstaaten: **DE FR GB IT**

(56) Entgegenhaltungen:
**DE-A-2 257 997**
**DE-A-2 315 245**
**DE-A-2 338 076**
**DE-A-2 418 540**

**Die Akte enthält technische Angaben, die nach dem Eingang der Anmeldung eingereicht wurden und die nicht in dieser Patentschrift enthalten sind.**

(73) Patentinhaber: **CIBA- GEIGY S.p.A., Strada Statale 233- Km. 20,5, Origgio (IT)**
(84) Benannte Vertragsstaaten: **IT**

(73) Patentinhaber: **CIBA- GEIGY AG, Klybeckstrasse 141, CH- 4002 Basel (CH)**
(84) Benannte Vertragsstaaten: **DE FR GB**

(72) Erfinder: **Cantatore, Giuseppe, Dr., Via Generale Planelli, 68, I-70032 Bitonto (Bari) (IT)**
Erfinder: **Borzatta, Valerio, Dr., Via Novelli 2, Bologna (IT)**

(74) Vertreter: **Stamm, Otto, Patentabteilung der CIBA-GEIGY AG Postfach, CH- 4002 Basel (CH)**

**EP 0 158 598 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die vorliegende Erfindung betrifft neue Piperidinverbindungen und ihre Verwendung als Stabilisatoren von organischem Material, insbesondere von synthetischen Polymeren gegen die Einwirkung von Licht, Wärme und Sauerstoff.

Zur Verbesserung der Lichtbeständigkeit von Polymeren werden verschiedene Stabilisatoren vorgeschlagen, die sich durch ihre gute lichtstabilisierende Wirkung auszeichnen. So werden z. B in der deutschen Auslegeschrift 1 929 928 Piperidylester von aliphatischen und aromatischen Carbonsäuren beschrieben.

Die neuen Verbindungen besitzen die allgemeine Formel (I)

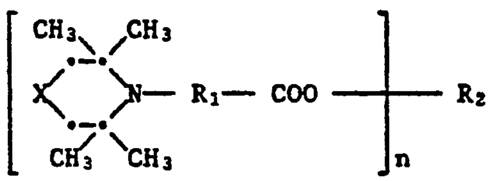
(I),

worin $R_1$ $C_1$-$C_{12}$-Alkylen ist, X Methylen, eine Gruppe FIG FIG, in welcher $R_3$ Wasserstoff, $C_1$-$C_{18}$-Alkyl, $C_3$-$C_{12}$-Alkenyl, $C_7$-$C_{18}$-Aralkyl oder $C_1$-$C_{12}$- Acyl ist, oder X ferner

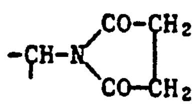
, eine Gruppe
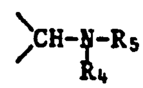
,

worin $R_4$ Wasserstoff, unsubstituiertes oder durch $C_1$-$C_{18}$-Alkoxy oder $C_2$-$C_{18}$-Dialkylamino substituiertes $C_1$-$C_{18}$-Alkyl oder $R_4$ ferner $C_5$-$C_{18}$-Cycloalkyl, $C_3$-$C_{18}$-Alkenyl oder $C_7$-$C_{18}$-Aralkyl ist und $R_5$ $C_1$-$C_{12}$-Acyl oder eine Gruppe -$COOR_6$ ist, in welcher $R_6$ $C_1$-$C_{12}$-Alkyl oder $C_3$-$C_{12}$-Alkenyl ist, oder X eine Gruppe

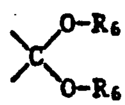

ist, worin $R_6$ die oben angegebene Bedeutung hat, oder X eine Gruppe der Formel (II) ist

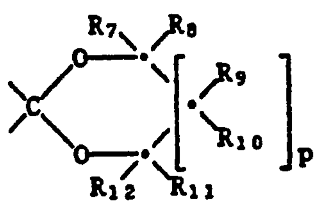
(II),

in welcher die Reste $R_7$ bis $R_{12}$ unabhängig voneinander Wasserstoff oder $C_1$-$C_4$-Alkyl sind und p 0 oder 1 ist, n 1 oder 2 ist, und wenn n = 1 ist, $R_2$ eine Gruppe der Formel (III) oder (IV) ist

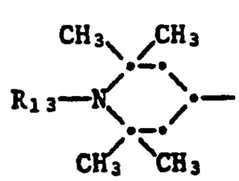
(III)

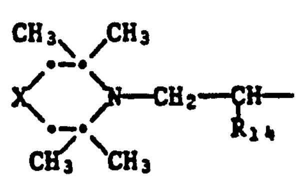
(IV)

worin $R_{13}$ Wasserstoff, 0·. Cyanmethyl, $C_1$-$C_{12}$-Alkyl, $C_3$-$C_{12}$-Alkenyl, $C_3$-$C_{12}$-Alkinyl, $C_7$-$C_{12}$-Aralkyl oder $C_1$-$C_{12}$-Acyl ist, X die oben angegebene Bedeutung hat und $R_{14}$ Wasserstoff, $C_1$-$C_6$-Alkyl oder $C_6$-$C_{12}$-Aryl ist, oder, wenn n = 2 ist, $R_2$ eine Gruppe der Formel (V) ist

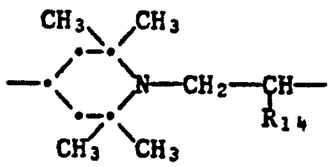 (V),

worin $R_{14}$ die oben angegebene Bedeutung hat.

Die folgenden Beispiele sollen die oben angegebenen Reste erläutern ohne sie einzuschränken:

Für $R_1$: Methylen, Ethylen, Propylen, Butylen, Pentamethylen, Hexamethylen, Octamethylen, Decamethylen und Dodecamethylen.

Für $R_3$: Wasserstoff, Methyl, Ethyl, Propyl, Butyl, Pentyl, Hexyl, Octyl, 2-Ethylhexyl, Decyl, Dodecyl, Tetradecyl, Hexadecyl, Octadecyl, Allyl, Methallyl, But-2-enyl, Hex-2-enyl, Undec-10-enyl, Benzyl, Methylbenzyl, t-Butylbenzyl, Hydroxybenzyl, 3,5-Di-t-butyl-4-hydroxybenzyl, Acetyl, Propionyl, Butyryl, Caproyl, Lauroyl und Benzoyl.

Für $R_4$: Wasserstoff, Methyl, Ethyl, Propyl, Butyl, Hexyl, Octyl, Decyl, Dodecyl, Tetradecyl, Hexadecyl, Octadecyl, 2-Methoxyethyl, 2-Ethoxyethyl, 3-Methoxypropyl, 3-Ethoxypropyl, 3-Butoxypropyl, 3-Octoxypropyl, Cyclohexyl, Methylcyclohexyl, Trimethylcyclohexyl, Cyclooctyl, Cyclododecyl, Allyl, Methallyl, But-2-enyl, Hex-2-enyl, Undec-10-enyl, Oleyl, Benzyl, Methylbenzyl, t-Butylbenzyl, Hydroxybenzyl und 3,5-Di-t-butyl-4-hydroxybenzyl.

Für $R_5$: Acetyl, Propionyl, Butyryl, Caproyl, Lauroyl und Benzoyl.

Für $R_6$: Methyl, Ethyl, Propyl, Butyl, Hexyl, Octyl, Decyl und Dodecyl, Allyl, But-2-enyl, Hex-3-enyl.

Für $R_7$ bis $R_{12}$: Wasserstoff, Methyl, Ethyl, Propyl und Butyl.

Für $R_{13}$: Wasserstoff, Cyanmethyl, O·, Methyl, Ethyl, Propyl, Butyl, Hexyl, Octyl, Decyl, Dodecyl, Allyl, Methallyl, But-2-enyl, Hex-2-enyl, Undec-10-enyl, Propargyl, Benzyl, Methylbenzyl, t-Butylbenzyl, Hydroxybenzyl, Acetyl, Propionyl, Butyryl, Caproyl, Lauroyl, Benzoyl, Acryloyl, Methacryloyl und Crotonyl.

Für $R_{14}$: Wasserstoff, Methyl, Ethyl, Propyl, Butyl, Phenyl, Methylphenyl, Dimethylphenyl und t-Butylphenyl.

Bevorzugt sind Verbindungen der Formel (I), worin $R_1$ $C_1$-$C_6$-Alkylen ist,

X Methylen, eine Gruppe 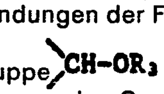 in welcher $R_3$ Wasserstoff, $C_1$-$C_{12}$-Alkyl, $C_3$-$C_6$-Alkenyl, $C_7$-$C_9$-Aralkyl oder $C_1$-$C_6$-Acyl ist, oder X ferner eine Gruppe 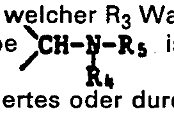 ist,

in welcher $R_4$ Wasserstoff, unsubstituiertes oder durch $C_1$-$C_{12}$-Alkoxy oder $C_2$-$C_{12}$-Dialkylamino substituiertes $C_1$-$C_{12}$-Alkyl oder $R_4$ ferner $C_6$-$C_9$-Cycloalkyl, $C_3$-$C_6$-Alkenyl oder $C_7$-$C_9$-Aralkyl ist und $R_5$ $C_1$-$C_6$-Acyl oder eine Gruppe -COOR$_6$ ist, worin $R_6$ $C_1$-$C_4$-Alkyl ist, oder X eine Gruppe der Formel (II) ist, in welcher $R_7$ bis $R_{12}$ Wasserstoff, Methyl oder Ethyl sind, n 1 oder 2 ist, wenn n. 1 ist, $R_2$ eine Gruppe der Formel (III) oder (IV) ist, worin $R_{13}$ Wasserstoff, $C_1$-$C_6$-Alkyl, $C_3$-$C_6$-Alkenyl, $C_3$-$C_6$-Alkinyl, Benzyl oder $C_1$-$C_6$-Acyl ist, X die oben angegebene Bedeutung hat und $R_{14}$ Wasserstoff, $C_1$-$C_4$-Alkyl oder Phenyl ist, und wenn $n = 2$ ist, $R_2$ eine Gruppe der Formel (V) ist, worin $R_{14}$ die oben angegebene Bedeutung hat.

Besonders bevorzugt sind Verbindungen der Formel (I), worin $R_1$ Methylen ist, X Methylen,

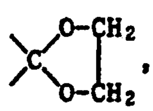, eine Gruppe 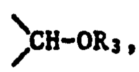,

worin $R_3$ $C_1$-$C_8$-Alkyl oder Allyl ist, oder X ferner eine Gruppe

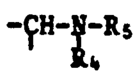 ist, in welcher $R_4$ $C_1$-$C_4$ Alkyl und $R_5$ $C_2$-$C_4$-Acyl ist,

n 1 oder 2 ist und, wenn $n = 1$ ist, $R_2$ eine Gruppe der Formel (III) oder (IV) ist, worin $R_{13}$ Wasserstoff oder Methyl ist, X die oben angegebene Bedeutung hat und $R_{14}$ Wasserstoff oder Methyl ist, und wenn $n = 2$ ist, $R_2$ eine Gruppe der Formel (V) ist, worin $R_{14}$ die oben angegebene Bedeutung hat.

Von besonderem Interesse sind Verbindungen der Formel (I), worin $R_1$ Methylen oder Propylen ist, X Methylen,

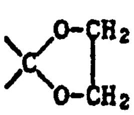 eine Gruppe 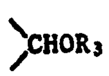, in welcher

$R_3$ n-Propyl, n-Butyl, n-Octyl oder Allyl ist oder X eine Gruppe

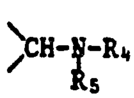 ist,

in welcher $R_4$ Ethyl oder n-Butyl ist und $R_5$ Acetyl oder $-COOC_2H_5$ ist, n 1 oder 2 ist, und, wenn n = 1 ist, $R_2$ eine Gruppe der Formel (III) oder (IV) ist, in welcher $R_{13}$ Wasserstoff oder Methyl ist,

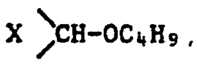 , ist

und $R_{14}$ Wasserstoff ist, und, wenn n = 2 ist, $R_2$ eine Gruppe der Formel (V) ist, in welcher $R_{14}$ Wasserstoff oder Methyl ist.

Die Verbindungen der Formel (I) können durch Umsetzen einer Verbindung der Formel (VI)

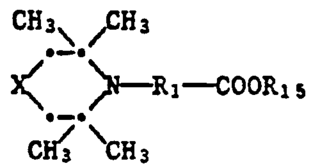 (VI),

worin X und $R_1$ die oben angegebene Bedeutung haben und $R_{15}$ $C_1$-$C_8$-Alkyl, bevorzugt $C_1$-$C_4$-Alkyl ist, mit einer Verbindung der Formel (VII) oder (VIII), wenn n = 1 ist,

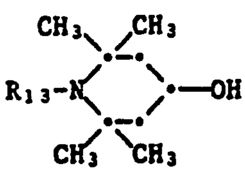

(VII)

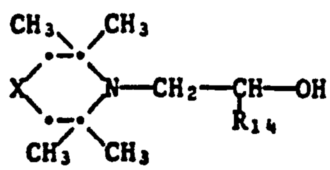

(VIII)

worin X $R_{13}$ und $R_{14}$ die oben angegebene Bedeutung haben, oder, wenn n = 2 ist mit einer Verbindung der Formel (IX)

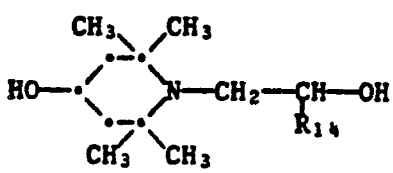 (IX)

worin $R_{14}$ die oben angegebene Bedeutung hat.

Die Reaktion kann mit oder ohne Lösungsmittel bei Temperaturen zwischen 100° und 250°C, bevorzugt zwischen 150° und 220°C, in Gegenwart eines geeigneten Umesterungskatalysators ablaufen.

Wird die Umsetzung mit Zusatz eines Lösungsmittels durchgeführt, so kann dieses z. B. aus folgender Gruppe inerter Lösungsmittel ausgewählt werden:

Toluol, Xylol, Trimethylbenzyl, Ethylbenzol, Tetralin, Decalin und ein aliphatischer Kohlenwasserstoff mit einem Siedepunkt zwischen 100° und 220°C. Der bei der Reaktion freiwerdende Alkohol wird abdestilliert.

Geeignete Umesterungskatalysatoren sind Alkalimetalle oder deren Hydride, Amide, Alkylderivate oder Alkoholate und ferner metallorganische Verbindungen, wie Tetraisopropoxytitan, Tetrabutoxytitan und Triisopropoxyaluminium.

Wenn n = 1 ist, kann das molare Verhältnis der Verbindung der Formel (VI) zur Verbindung der Formel (VII)

oder (VIII) stöchiometrisch sein, d. h. 1 : 1, aber es ist vorteilhaft, einen Überschuss, z. B. bis zu 20 % des theoretischen Wertes, von einem der beiden Reagentien einzusetzen.

Wenn n = 2 ist, kann das molare Verhältnis der Verbindung der Formel (VI) zur Verbindung der Formel (IX) stöchiometrisch sein, d. h. 2 : 1, aber bevorzugt wird ein Überschuss, z. B. bis zu 20 % des theoretischen Wertes, von Verbindungen der Formel (VI) eingesetzt.

Verbindungen der Formel (VI) können in Übereinstimmung mit bekannten Verfahren durch Umsetzung einer Verbindung der Formel (X)

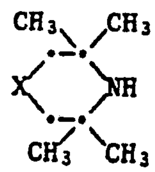

(X),

worin X die oben angegebene Bedeutung hat, mit einem Halogenester der Formel (XI)

$$Y - R_1 - COOR_{15} \qquad (XI),$$

worin Y Halogen ist, bevorzugt Chlor oder Brom, und $R_{15}$ die oben angegebene Bedeutung hat, erhalten werden.

Die Verbindungen der Formeln (VII), (VIII) und (IX) sind bekannt.

Zur Veranschaulichung wird die Herstellung von 1-(Ethoxycarbonyl-methyl)-2,2,6,6-tetramethylpiperidin beschrieben.

Herstellung von 1-(Ethoxycarbonylmethyl)-2,2,6,6-tetramethyl-piperidin

20 g Kaliumjodid und 290 g (2,37 Mol) Chloressigsäurethylester werden zu 334 g (2,37 Mol) 2,2,6,6-Tetramethylpiperidin gegeben, welches in 650 ml Methyl-ethyl-keton gelöst ist.

Die so erhaltene Mischung wird unter Rückfluss 16 Stunden lang erhitzt. Nach dem Abkühlen werden 327 g (2,37 Mol) fein zermahlenes, wasserfreies Kaliumcarbonat hinzugefügt.

Die Mischung wird unter Rückfluss nochmals 16 Stunden lang erhitzt und nach dem Abkühlen gefiltert und im Vakuum eingeengt.

Der erhaltene Rückstand wird in 600 ml Methylenchlorid aufgenommen und die Lösung wird mit Wasser gewaschen. Die organische Lösung wird dann über Natriumsulfat getrocknet und anschliessend eingeengt. Der Rückstand wird destilliert. Die Ausbeute beträgt 430,7 g. Das Produkt hat einen Siedepunkt von 124 - 126°C bei 17 mmHg.

Analog wird das Verfahren für die Herstellung der folgenden Verbindungen der Formel (VI) wiederholt, welche für die Synthese der Verbindungen der Formel (I) verwendet werden:

**Tabelle 1**

| Verbindung der Formel (VI) | Siedepunkt (°C/mmHg) |
|---|---|
| 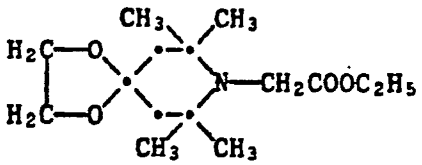 | 153-155/7 |
| 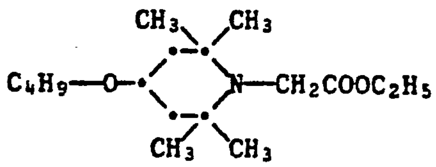 | 171-173/20 |
| 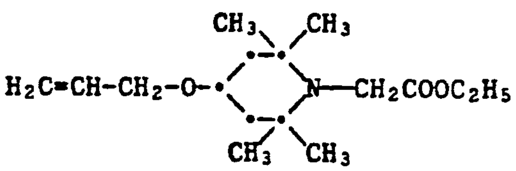 | 162-164/15 |
| 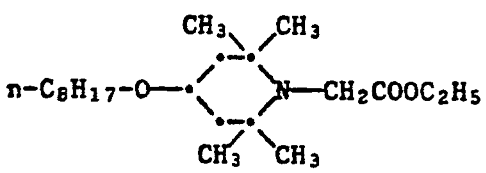 | 184-186/2 |
| 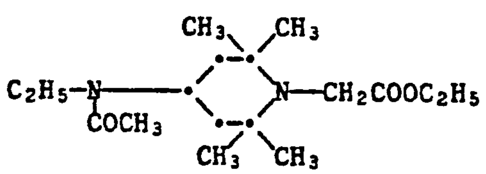 | 172-174/1 |
| 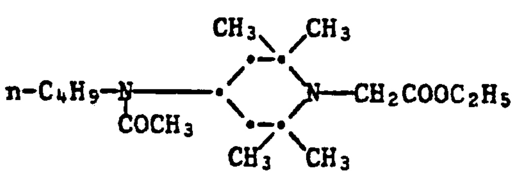 | 220-221/1.5 |

Einige Herstellungsbeispiele der Verbindungen der Formel (I) werden weiter unten beschrieben, um die vorliegende Erfindung deutlicher zu veranschaulichen; diese Beispiele werden nur zur Veranschaulichung gegeben und bedeuten keinerlei Einschränkung.

**Beispiel 1:**

Herstellung von

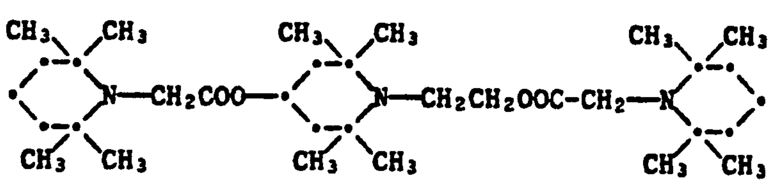

300 g (1,32 Mol) 1-(Ethoxycarbonylmethyl)-2,2,6,6-tetramethylpiperidin und 5,7 g Tetraisopropoxytitan werden zu 120,6 g (0,6 Mol) 1-(2-Hydroxyethyl)-4-hydroxy-2,2,6,6-tetramethylpiperidin gegeben.

Die Mischung wird unter einem Stickstoffstrom 3 Stunden bei einer Temperatur von 160 - 170°C erhitzt und 4 Stunden bei 190 - 200°C. Das während der Reaktion freiwerdende Ethanol (70,8 ml) wird entfernt und das Reaktionsgemisch wird schliesslich noch 1 Stunde lang bei 160 - 170°C im Vakuum (20 mmHg) erhitzt.

Das Reaktionsgemisch wird abgekühlt und 180 ml Petrolether (Siedepunkt 60 - 80°C) werden hinzugefügt. Der erhaltene Niederschlag wird durch Filtration abgetrennt und aus Petrolether (Siedepunkt 60 - 80°C) umkristallisiert.

Man erhält eine Verbindung der Formel

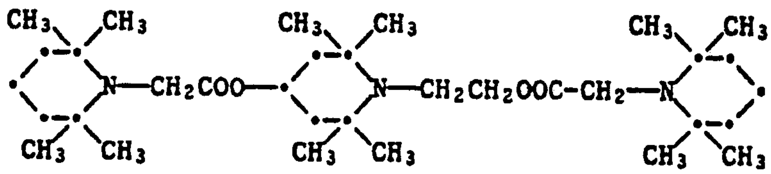

Das erhaltene Produkt hat einen Schmelzpunkt bei 143 - 144°C.
Elementaranalyse von $C_{33}H_{61}N_3O_4$:

| | | | |
|---|---|---|---|
| berechnet: | C 70,30 % | H 10,90 % | N 7,45 % |
| gefunden: | C 70,27 % | H 10,83 % | N 7,40 %. |

**Beispiele 2 - 21:**

Das im Beispiel 1 beschriebene Verfahren wird für die Herstellung der folgenden Verbindungen der Formel (I) unter Verwendung der entsprechenden Zwischenprodukte der Formel (VII), (VIII) oder (IX) wiederholt.

Tabelle 2

| Beispiel Nr. | X | $R_1$ | $R_2$ | n | Schmelzpunkt (°C) Siedepunkt (°c/mmHg) |
|---|---|---|---|---|---|
| 2 | 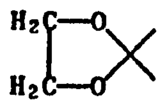 | $-CH_2-$ | 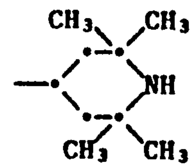 | 1 | 135-136 |
| 3 | 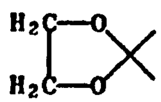 | $-CH_2-$ | 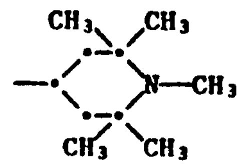 | 1 | 113-114 |
| 4 | 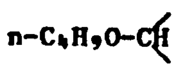 | $-CH_2-$ | 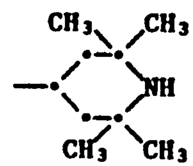 | 1 | 200-202/1.5 |
| 5 | 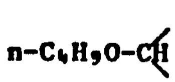 | $-CH_2-$ | 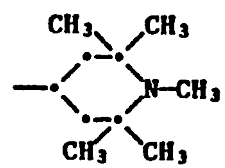 | 1 | 202-203/0.5 |

Tabelle 2

| Beispiel Nr. | X | $R_1$ | $R_2$ | n | Schmelzpunkt (°C) Siedepunkt (°c/mmHg) |
|---|---|---|---|---|---|
| 6 | $H_2C=CH-CH_2-O-CH$ | $-CH_2-$ | 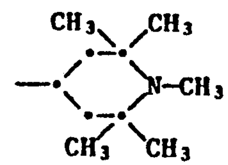 | 1 | 193-194/0.4 |
| 7 | $n-C_8H_{17}-O-CH$ | $-CH_2-$ | 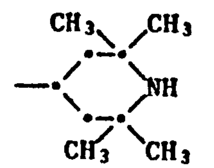 | 1 | 219-220/0.5 |
| 8 | $n-C_8H_{17}-O-CH$ | $-CH_2-$ | 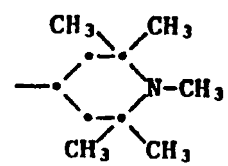 | 1 | 225-226/0.4 |
| 9 | $H_5C_2-N-CH$ $COCH_3$ | $-CH_2-$ | 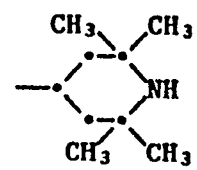 | 1 | 132-133 |

<u>Tabelle 2</u> (Fortsetzung)

| Beispiel Nr. | X | $R_1$ | $R_2$ | n | Schmelzpunkt (°C) Siedepunkt (°c/mmHg) |
|---|---|---|---|---|---|
| 10 | 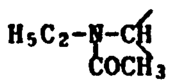 | $-CH_2-$ | 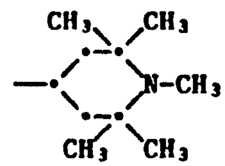 | 1 | 113-114 |
| 11 | 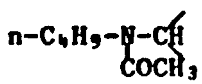 | $-CH_2-$ | 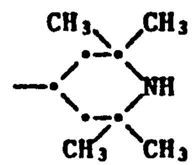 | 1 | 153-154 |
| 12 | 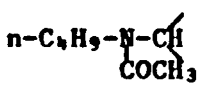 | $-CH_2-$ | 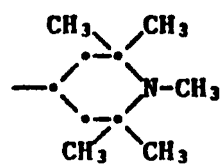 | 1 | 244-246/0.8 |
| 13 | 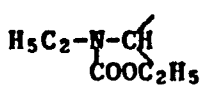 | $-CH_2-$ | 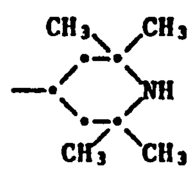 | 1 | Harz |

Tabelle 2

| Beispiel Nr. | X | $R_1$ | $R_2$ | n | Schmelzpunkt (°C) Siedepunkt (°c/mmHg) |
|---|---|---|---|---|---|
| 14 | 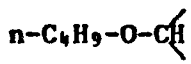 | $-CH_2-$ | 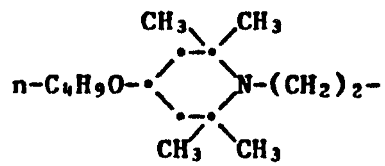 | 1 | 233-235/0.4 |
| 15 | 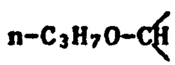 | $-(CH_2)_3-$ | 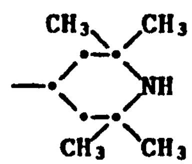 | 1 | 215-216/0.2 |
| 16 | 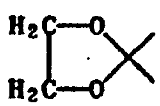 | $-CH_2-$ | 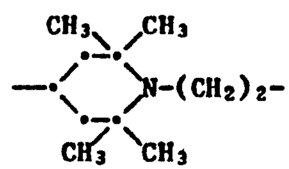 | 2 | 173-174 |
| 17 | 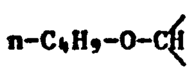 | $-CH_2-$ | 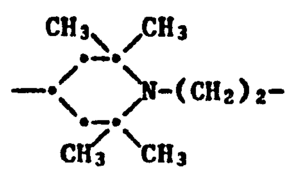 | 2 | 118-119 |

Tabelle 2

| Beispiel Nr. | X | $R_1$ | $R_2$ | n | Schmelzpunkt (°C) Siedepunkt (°c/mmHg) |
|---|---|---|---|---|---|
| 18 | 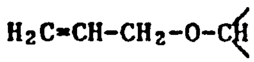 | $-CH_2-$ | 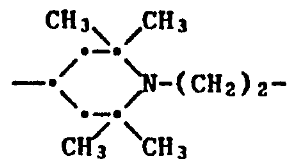 | 2 | 102-103 |
| 19 | 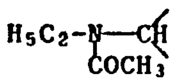 | $-CH_2-$ | 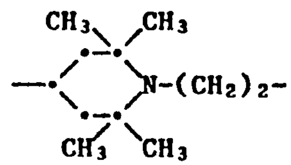 | 2 | 186-187 |
| 20 | 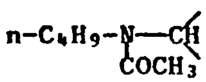 | $-CH_2-$ | 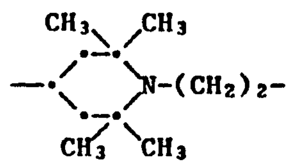 | 2 | 138-139 |
| 21 | 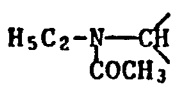 | $-CH_2-$ | 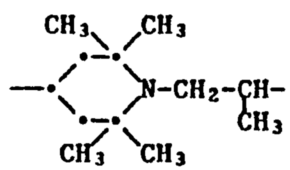 | 2 | 137-138 |

Wie anfangs erwähnt, sind die Verbindungen der Formel (I) sehr wirksam bei der Verbesserung der Licht-, Wärme- und Oxydationsbeständigkeit von organischem Material Wie beispielsweise Polyethylen hoher und niedriger Dichte, Polypropylen, Ethylen/Propylencopolymeren, Ethylen/Vinylacetatcopolymeren, Polybutadien, Polyisopren, Polystyrol, Butadien/Styrolcopolymeren, Polymeren und Copolymeren von Vinylchlorid und Vinylidenchlorid, Polyoxymethylen, Polyurethanen, gesättigten und ungesättigten Polyestern, Polyamiden, Polycarbonaten, Polyacrylaten, Alkydharzen und Epoxidharzen.

Die Verbindungen der Formel (I) können mit organischem Material, z. B. mit synthetischen Polymeren, in verschiedenen Anteilen gemischt werden, die von der Art des Polymeren, dem Endzweck und der Gegenwart weiterer Zusatzstoffe abhängen. Im allgemeinen setzt man zweckmässig 0,01 bis 5 Gew.-%, vorzugsweise 0,05 bis 1 Gew.-%, der Verbindungen der Formel (I), bezogen suf das Gewicht der Polymeren ein.

Die Verbindungen der Formel (I) können nach verschiedenen Verfahren in die polymeren Materialien eingearbeitet werden, wie durch trockenes Einmischen in Pulverform oder nasses Einmischen in Form einer Lösung oder Suspension oder auch in Form eines konzentrierten Vorgemisches; das Polymer lässt sich dabei in Form von Pulver, Granulat, Lösung, Suspension oder Latex einsetzen. Das mit den Produkten der Formel (I) stabilisierte organische Material lässt sich zur Herstellung von Formkörpern, Folien, Bändern, Fasern, Endlosfäden, Lacken usw. verwenden.

Gegebenenfalls kann man den Mischungen von Verbindungen der Formel (I) mit den Polymeren Weitere Zusatzstoffe beigeben, wie beispielsweise Antioxidantien, UV-absorbierende Stoffe, Nickelstabilisatoren, Pigmente, Füllstoffe, Weichmacher, Antistatika, Flammschutzmittel, Schmierstoffe, Korrosionshemmstoffe sowie Metalldesaktivatoren. Beispiele für in Mischung mit Verbindungen der Formel (I) verwendbare Zusatzstoffe sind insbesondere:

1. Antioxidantien

1.1. Alkylierte Monophenole

2,6-Di-tert.butyl-4-methylphenol
2-Tert.butyl-4,6-dimethylphenol
2,6-Di-tert.butyl-4-ethylphenol
2,6-Di-tert.butyl-4-n-butylphenol
2,6-Di-tert.butyl-4-i-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert.butyl-4-methoxymethylphenol

1.2. Alkylierte Hydrochinone

2,6-Di-tert.butyl-4-methoxyphenol
2,5-Di-tert.butyl-hydrochinon
2,5-Di-tert.amyl-hydrochinon
2,6-Diphenyl-4-octadecyloxyphenol

1.3. Hydroxylierte Thiodiphenylether

2,2'-Thio-bis-(6-tert.butyl-4-methylphenol)
2,2'-Thio-bis-(4-octylphenol)
4,4'-Thio-bis-(6-tert.butyl-3-methylphenol)
4,4'-Thio-bis-(6-tert.butyl-2-methylphenol)

1.4. Alkyliden-Bisphenole

2,2'-Methylen-bis-(6-tert.butyl-4-methylphenol)
2,2'-Methylen-bis-(6-tert.butyl-4-ethylphenol)
2,2'-Methylen-bis-(4-methyl-6-(α-methylcyclohexyl)-phenol)
2,2'-Methylen-bis-(4-methyl-6-cyclohexylphenol)
2,2'-Methylen-bis-(6-nonyl-4-methylphenol)
2,2'-Methylen-bis-(4,6-di-tert.butylphenol)
2,2'-Ethyliden-bis-(4,6-di-tert.butylphenol)
2,2'-Ethyliden-bis-(6-tert.butyl-4-isobutylphenol)
2,2'-Methylen-bis-[6-(α-methylbenzyl)-4-nonylphenol]
2,2'-Methylen-bis-[6-(α,α-dimethylbenzyl)-4-nonylphenol]
4,4'-Methylen-bis-(2,6-di-tert.butylphenol)
4,4'-Methylen-bis-(6-tert.butyl-2-methylphenol)
1,1-Bis-(5-tert.butyl-4-hydroxy-2-methylphenyl)-butan
2,6-Di-(3-tert.butyl-5-methyl-2-hydroxybenzyl)-4-methylphenol
1,1,3-Tris-(5-tert.butyl-4-hydroxy-2-methylphenyl)-butan
1,1-Bis-(5-tert.butyl-4-hydroxy-2-methylphenyl)-3-n-dodecylmercaptobutan
Ethylenglycol-bis-[3,3-bis-(3'-tert.butyl-4'-hydroxyphenyl)-butyrat]
Di-(3-tert.butyl-4-hydroxy-5-methylphenyl)-dicyclopentadien

Di-[2-(3'-tert.butyl-2'-hydroxy-5'-methyl-benzyl)-6-tert.butyl-4-methyl-phenyl]-terephthalat.

1.5 Benzylverbindungen
1,3,5-Tri-(3,5-di-tert.butyl-4-hydroxybenzyl)-2,4,6-trimethylbenzol
Di-(3,5-di-tert.butyl-4-hydroxybenzyl)-sulfid
3,5-di-tert.butyl-4-hydroxybenzyl-mercaptoessigsäure-isooctylester
Bis-(4-tert.butyl-3-hydroxy-2,6-dimethylbenzyl)-dithiol-terephthalat
1,3,5-Tris-(3,5-di-tert.butyl-4-hydroxybenzyl)-isocyanurat
1,3,5-Tris-(4-tert.butyl-3-hydroxy-2,6-dimethylbenzyl)-isocyanurat
3,5-Di-tert.butyl-4-hydroxybenzyl-phosphonsäure-dioctadecylester
3,5-Di-tert.butyl-4-hydroxybenzyl-phosphonsäure-monoethylester
Calcium-salz.

1.6. Acylaminophenole
4-Hydroxy-laurinsäureanilid
4-Hydroxy-stearinsäureanilid
2,4-Bis-octylmercapto-6-(3,5-di-tert.butyl-4-hydroxyanilino)-s-triazin
N-(3,5-di-tert.butyl-4-hydroxyphenyl)-carbaminsäureoctylester.

1.7. Ester der β-(3,5-Di-tert.butyl-4-hydroxyphenyl)-propionsäure
mit ein- oder mehrwertigen Alkoholen, wie z. B. mit

| | |
|---|---|
| Methanol | Diethylenglycol |
| Octadecanol | Triethylenglycol |
| 1,6-Hexandiol | Pentaerythrit |
| Heopentylglycol | Tris-hydroxyethyl-isocyanurat |
| Thiodiethylenglycol | Di-hydroxyethyl-oxalsäurediamid |

1.8. Ester der β-(5-tert.butyl-4-hydroxy-3-methylphenyl)-propionsäure
mit ein- oder mehrwertigen Alkoholen, wie z. B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |

1.9. Amide der β-(3,5-Di-tert.butyl-4-hydroxyphenyl)-propionsäure,

wie z. B.
N,N'-Di-(3,5-di-tert.butyl-4-hydroxyphenylpropionyl)-hexamethylendiamin
N,N'-Di-(3,5-di-tert.butyl-4-hydroxyphenylpropionyl)-trimethylendiamin
N,N'-Di-(3,5-di-tert.butyl-4-hydroxyphenylpropionyl)-hydrazin

2. UV-Absorber und Lichtschutzmittel

2.1 2-(2'-Hydroxyphenyl)-benztriazole,
wie z. B. das
5'-Methyl-, 3',5'-Di-tert.butyl-, 5'-Tert.butyl-, 5'-(1,1,3,3-Tetramethyl-butyl)-, 5-Chlor-3',5'-di-tert.butyl-, 5-Chlor-3'-tert.butyl-5'-methyl-, 3'-sec.Butyl-5'-tert.butyl, 4'-Octoxy-, 3',5'-Di-tert.amyl-, 3',5'-Bis-(α,α-dimethylbenzyl)-Derivat.

2.2. 2-Hydroxybenzophenone,
wie z. B. das
4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren,
wie z. B.
4-Tert.butyl-phenylsalicylat, Phenylsalicylat, Octylphenylsalicylat, Dibenzoylresorcin. Bis-(4-tert.butylbenzoyl)-resorcin, Benzoylresorcin, 3,5-Di-tert.butyl-4-hydroxybenzoesäure-2,4-di-tert.butylphenylester, 3,5-Di-tert.butyl-4-hydroxybenzoesäurehexa-decylester.

2.4. Acrylate,
wie z. B.
α-Cyan-β,β-diphenylacrylsäure-ethylester bzw. -isooctylester, α-Carbomethoxy-zimtsäuremethylester, α-

Cyano-β-methyl-p-methoxy-zimtsäuremethylester bzw. -butylester, α-Carbomethoxy-p-methoxy-zimtsäuremethylester, N-(β-Carbomethoxy-β-cyanovinyl)-2-methyl-indolin.

2.5. Nickelverbindungen,
wie z. B. Nickelkomplexe des
2,2'-Thio-bis-(4-(1,1,3,3-tetramethylbutyl)-phenols], wie der 1 : 1- oder der 1 : 2-Komplex, gegebenenfalls mit zusätzlichen Liganden wie n-Butylamin, Triethanolamin oder N-Cyclohexyl-diethanolamin, Nickeldibutyldithiocarbamat, Nickelsalze von 4-Hydroxy-3,5-di-tert.butylbenzyl-phosphonsäure-monoalkylestern wie vom Methyl- oder Ethylester, Nickelkomplexe von Ketoximen wie von 2-Hydroxy-4-methyl-phenyl-undecylketonoxim, Nickelkomplexe des 1-Phenyl-4-lauroyl-5-hydroxy-pyra-zols, gegebenenfalls mit zusätzlichen Liganden.

2.6. Sterisch gehinderte Amine,
wie z. B.
Bis-(2,2,6,6-tetramethyl-piperidyl)-sebacat
Bis-(1,2,2,6,6-pentamethylpiperidyl)-sebacat n-Butyl-3,5-di-tert.butyl-4-hydroxybenzyl-malonsäure-bis-(1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-(2,2,6,6-Tetramethyl-4-piperidyl)-hexamethylendiamin und 4-tert.Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3,4-butantetracarbon-säure, 1,1'-(1 2-Ethandiyl)-bis-(3,3,5,5-tetramethyl-piperazinon).

2.7. Oxalsäurediamide,
wie z. B.
4,4'-Di-octyloxy-oxanilid, 2,2'-Di-octyloxy-5,5'-di-tert.butyl-oxanilid, 2,2'-Di-dodecyloxy-5,5'-di-tert.butyl-oxanilid, 2-Ethoxy-2'-ethyloxanilid, N,N'-Bis-(3-dimethylaminopropyl)-oxalamid, 2-Ethoxy-5-tert.butyl-2'-ethyl-oxanilid und dessen Gemisch mit 2-Ethoxy-2'-ethyl-5,4'-di-tert.butyl-oxanilid, Gemische von ortho-und para-Methoxy- sowie von o- und p-Ethoxy-di-substituierte Oxaniliden.

3. Metalldesaktivatoren,
wie z. B.
N,N'-Diphenyloxalsäurediamid, N-Salicylal-N'-salicyloylhydrazin, N,N'-Bis-salicyloylhydrazin, N,N'-Bis-(3,5-di-tert.butyl-4-hydroxyphenylpropionyl)-hydrazin, 3-Salicyloylamino-1,2,4-triazol, Bis-benzylidenoxalsäuredihydrazid.

4. Phosphite und Phosphonite,
wie z. B.
Triphenylphosphit, Diphenylalkylphosphite, Phenyldialkylphosphite, Tri-(nonylphenyl)-phosphit, Trilaurylphosphit, Trioctadecylphosphit, Distearyl-pentaerythritdi phosphit, Tris-(2,4-di-tert.butylphenyl)-phosphit, Diisodecylpentaerythrit-diphosphit, Di-(2,4-di-tert.butylphenyl)-pentaerythritdi-phosphit, Tristearyl-sorbit-triphosphit, Tetrakis-(2,4-di-tert.butylphenyl)-4,4'-biphenylen-diphosphonit.

5. Peroxidzerstörende Verbindungen,
wie z. B.
Ester der β-Thio-dipropionsäure, beispielsweise der Lauryl-, Stearyl-, Myristyl- oder Tridecylester, Mercaptobenzimidazol, das Zinksalz des 2-Mercapto-benzimidazols, Zink-dibutyl-dithiocarbamat, Dioctadecyldisulfid, Pentaerythrit-tetrakis-(β-dodecylmercapto)-propionat.

6. Polyamidstabilisatoren,
wie z. B.
Kupfersalze in Kombination mit Jodiden und/oder Phosphorverbindungen und Salze des zweiwertigen Mangans.

7. Basische Co-Stabilisatoren,
wie z. B.
Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel,
wie z. B.
4-tert.Butylbenzoesäure, Adipinsäure, Diphenylessigsäure.

9. Füllstoffe und Verstärkungsmittel,
wie z. B.

Calciumcarbonat, Silikate, Glasfasern, Asbest, Talk, Kaolin, Glimmer, Bariumsulfat, Metalloxide und -hydroxide, Russ, Graphit.

10. Sonstige Zusätze,
wie z. B.
Weichmacher, Gleitmittel, Emulgatoren, Pigmente, Optische Aufheller, Flammschutzmittel, Antistatika, Treibmittel.

**Beispiel 22:**

2 g von jeder der in Tabelle 3 angegebenen Verbindungen, 1 g Pentaerythrit-tetrakis-3-(3,5-di-t-butyl-4-hydroxyphenyl)-propionat (Antioxidans) und 1 g Calciumstearat werden mit 1000 g Polypropylenpulver mit einem Schmelzindex von 3 (®Propathen HF 18, ein Produkt der Imperial Chemical Industies) innig vermischt.
Das erhaltene Gemisch wird dann bei einer Temperatur von 200 bis 230°C extrudiert und zu Granulat verarbeitet, aus dem man Bänder einer Dicke von 40 µm und einer Breite von 3 mm herstellt. Die Arbeitsbedingungen sind:

| | |
|---|---|
| Extrudertemperatur: | 230 - 240°C |
| Kopftemperatur: | 240°C |
| Streckverhältnis: | 1 : 6 |

Die so erhaltenen Bänder werden auf einer weissen Pappe befestigt den Bedingungen in einem Weather-Ometer 65 WR (ASTM G 27 - 70) ausgesetzt. Die Schwarztafeltemperatur beträgt 63°C. Nach unterschiedlich langen Belichtungszeiten werden Proben entnommen, an denen man die verbleibende Zugfestigkeit mittels eines Tensometers mit konstanter Geschwindigkeit misst. Dann wird die zum Halbieren der ursprünglichen Zugfestigkeit erforderliche Belichtungszeit ($T_{50}$) in Stunden berechnet. Zum Vergleich wird eine Polypropylenfolie belichtet, die unter den oben angegebenen Bedingungen aber ohne Zugabe der erfindungsgemässen Verbindungen hergestellt wurde.
Die erhaltenen Resultate werden in Tabelle 3 wiedergegeben:

**Tabelle 3**

| Stabilisator | | $T_{50}$ (Stunden) |
|---|---|---|
| keiner | | 220 |
| Verbindung von Beispiel | 1 | 1960 |
| Verbindung von Beispiel | 2 | 2640 |
| Verbindung von Beispiel | 4 | 2990 |
| Verbindung von Beispiel | 5 | 2250 |
| Verbindung von Beispiel | 6 | 2830 |
| Verbindung von Beispiel | 7 | 1860 |
| Verbindung von Beispiel | 8 | 2550 |
| Verbindung von Beispiel | 9 | 2340 |
| Verbindung von Beispiel | 10 | 2320 |
| Verbindung von Beispiel | 11 | 2000 |
| Verbindung von Beispiel | 12 | 2290 |
| Verbindung von Beispiel | 18 | 2000 |
| Verbindung von Beispiel | 19 | 1890 |
| Verbindung von Beispiel | 21 | 1790 |

**Beispiel 23:**

1 g von jeder der in Tabelle 4 angegebenen Verbindungen, 1 g Pentaerythrit-tetrakis-(3,5-di-t-butyl-4-hydroxyphenyl)propionat, 1 g Calciumstearat, 1 g Phthalocyaninblau und 1000 g Polypropylenpulver mit einem Schmelzindex von 3 (®propathen HF 18, Produkt der Imperial Chemical Industries) werden innig vermischt.
Die erhaltenen Gemische werden bei einer Temperatur von 200, bis 220°C extrudiert und zu Granulaten verarbeitet, aus denen durch Extrusion bei 250°C 2 mm dicke Folien hergestellt werden.
Die erhaltenen Folien werden in einem Weather-Ometer 65 WR (ASTM G 27 - 70) bis zum wahrnehmbaren Beschlagen der Oberfläche (Kreidung) belichtet. Die Schwarztafeltemperatur beträgt 63°C.
Zum Vergleich wird eine Polypropylenfolie belichtet, die unter den oben angegebenen Bedingungen aber ohne Zugabe der erfindungsgemäßsen Verbindungen hergestellt wurde.

In Tabelle 4 wird die Belichtungszeit (in Stunden) angegeben, die bis zum Beginn der Kreidung erforderlich ist.

**Tabelle 4**

| Stabilisator | Zeit bis Kreidung (in Stunden) |
|---|---|
| keiner | 500 |
| Verbindung von Beispiel 1 | 2410 |
| Verbindung von Beispiel 3 | 2910 |
| Verbindung von Beispiel 4 | 3260 |
| Verbindung von Beispiel 5 | 3260 |
| Verbindung von Beispiel 7 | 3260 |
| Verbindung von Beispiel 8 | 3440 |
| Verbindung von Beispiel 9 | 3260 |
| Verbindung von Beispiel 10 | 3100 |
| Verbindung von Beispiel 11 | 3100 |
| Verbindung von Beispiel 12 | 3100 |
| Verbindung von Beispiel 16 | 2710 |
| Verbindung von Beispiel 17 | 2410 |

**Patentansprüche**

1. Eine Verbindung der allgemeinen Formel (I)

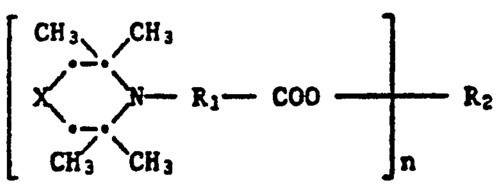 (I),

worin $R_1$ $C_1$-$C_{12}$-Alkylen ist, X Methylen, eine Gruppe 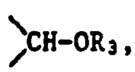,

in welcher $R_3$ Wasserstoff, $C_1$-$C_{18}$-Alkyl, $C_3$-$C_{12}$-Alkenyl, $C_7$-$C_{18}$-Aralkyl oder $C_1$-$C_{12}$-Acyl ist, oder X ferner

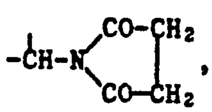, eine Gruppe 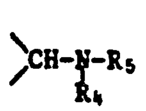,

worin $R_4$ Wasserstoff, unsubstituiertes oder durch $C_1$-$C_{18}$-Alkoxy oder $C_2$-$C_{18}$-Dialkylamino substituiertes $C_1$-$C_{18}$-Alkyl oder $R_4$ ferner $C_5$-$C_{18}$-Cycloalkyl, $C_3$-$C_{18}$-Alkenyl oder $C_7$-$C_{18}$-Aralkyl ist und $R_5$ $C_1$-$C_{12}$-Acyl oder eine Gruppe -$COOR_6$ ist, in welcher $R_6$ $C_1$-$C_{12}$-Alkyl oder $C_3$-$C_{12}$-Alkenyl ist, oder X eine Gruppe

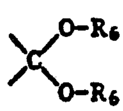

ist, worin $R_6$ die oben angegebene Bedeutung hat, oder X eine Gruppe der Formel (II) ist

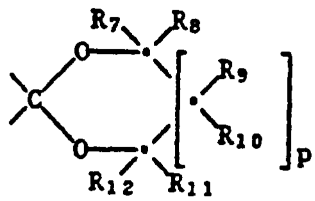

(II),

in welcher die Reste $R_7$ bis $R_{12}$ unabhängig voneinander Wasserstoff oder $C_1$-$C_4$-Alkyl sind und p 0 oder 1 ist, n 1 oder 2 ist und wenn n = 1 ist, $R_2$ eine Gruppe der Formel (III) oder (IV) ist

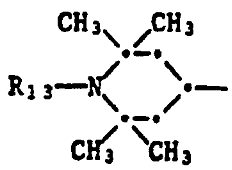

(III)

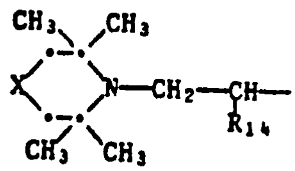

(IV)

worin $R_{13}$ Wasserstoff, O·, Cyanmethyl, $C_1$-$C_{12}$-Alkyl, $C_3$-$C_{12}$-Alkenyl, $C_3$-$C_{12}$-Alkinyl, $C_7$-$C_{12}$-Aralkyl oder $C_1$-$C_{12}$-Acyl ist, X die oben angegebene Bedeutung hat und $R_{14}$ Wasserstoff, $C_1$-$C_6$-Alkyl oder $C_6$-$C_{12}$-Aryl ist, oder, wenn n = 2 ist, $R_2$ eine Gruppe der Formel (V) ist

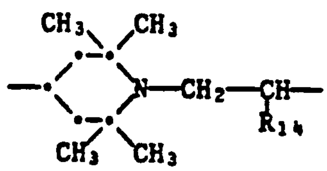

(V),

worin $R_{14}$ die oben angegebene Bedeutung hat.

2. Eine Verbindung der Formel (I) gemäss Anspruch 1, worin $R_1$ $C_1$-$C_6$-Alkylen ist, X Methylen, eine Gruppe 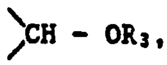, in welcher $R_3$ Wasserstoff, $C_1$-$C_{12}$-Alkyl, $C_3$-$C_6$-Alkenyl, $C_7$-$C_9$-Aralkyl oder $C_1$-$C_6$-Acyl ist, oder X ferner eine Gruppe

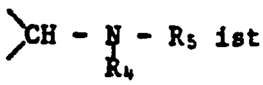 ist,

in welcher $R_4$ Wasserstoff, unsubstituiertes oder durch $C_1$-$C_{12}$-Alkoxy oder $C_2$-$C_{12}$-Dialkylamino substituiertes $C_1$-$C_{12}$-Alkyl oder $R_4$ ferner $C_6$-$C_9$-Cycloalkyl, $C_3$-$C_6$-Alkenyl oder $C_7$-$C_9$-Aralkyl ist und $R_5$ $C_1$-$C_6$-Acyl oder eine Gruppe -$COOR_6$ ist, worin $R_6$ $C_1$-$C_4$-Alkyl ist, oder X eine Gruppe der Formel (II) ist, in welcher $R_7$ bis $R_{12}$ Wasserstoff, Methyl oder Ethyl sind, n 1 oder 2 ist und, wenn n = 1 ist, $R_2$ eine Gruppe der Formel (III) oder (IV) ist, worin $R_{13}$ Wasserstoff, $C_1$-$C_6$-Alkyl, $C_3$-$C_6$-Alkenyl, $C_3$-$C_6$-Alkinyl, Benzyl oder $C_1$-$C_6$-Acyl ist, X die oben angegebene Bedeutung hat und $R_{14}$ Wasserstoff, $C_1$-$C_4$-Alkyl oder Phenyl ist, und wenn n = 2 ist, $R_2$ eine Gruppe der Formel (V) ist, worin $R_{14}$ die oben angegebene Bedeutung hat.

3. Eine Verbindung der Formel (I) gemäss Anspruch 1, worin $R_1$ Methylen ist, X Methylen,

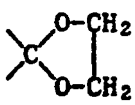, eine Gruppe 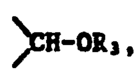, worin $R_3$ $C_1$-$C_8$-Alkyl oder Allyl

ist, oder X ferner eine Gruppe 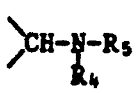 ist, in welcher

$R_4$ $C_1$-$C_4$-Alkyl und $R_5$ $C_2$-$C_4$-Acyl ist, n 1 oder 2 ist und, wenn n = 1 ist, $R_2$ eine Gruppe der Formel (III) oder (IV) ist, worin $R_{13}$ Wasserstoff oder Methyl ist, X die oben angegebene Bedeutung hat und $R_{14}$ Wasserstoff oder Methyl ist, und wenn n = 2 ist, $R_2$ eine Gruppe der Formel (V) ist, worin $R_{14}$ die oben angegebene Bedeutung hat.

4. Eine Verbindung der Formel (I) gemäss Anspruch 1, worin $R_1$ Methylen oder Propylen ist, X Methylen, 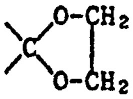, eine Gruppe 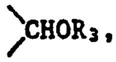, in welcher $R_3$ n-Propyl, n-Butyl, n-Octyl oder Allyl ist, oder X eine Gruppe 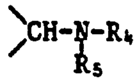 ist, in welcher $R_4$ Ethyl oder n-Butyl ist und $R_5$ Acetyl oder $-COOC_2H_5$ ist, n 1 oder 2 ist, und, wenn n = 1 ist, $R_2$ eine Gruppe der Formel (III) oder (IV) ist, in welcher $R_{13}$ Wasserstoff oder Methyl ist, X 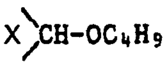 ist, und $R_{14}$ Wasserstoff ist, und, wenn n = 2 ist, $R_2$ eine Gruppe der Formel (V) ist, in welcher $R_{14}$ Wasserstoff oder Methyl ist.

5. Verbindungen gemäss Anspruch 1 mit den folgenden Formeln:

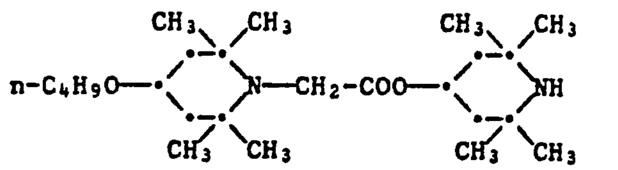

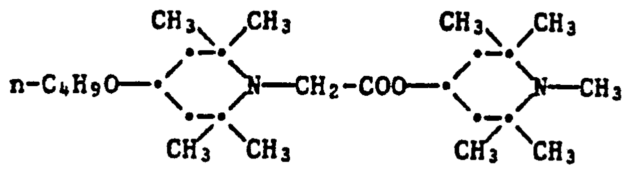

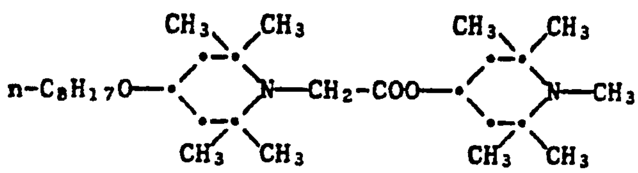

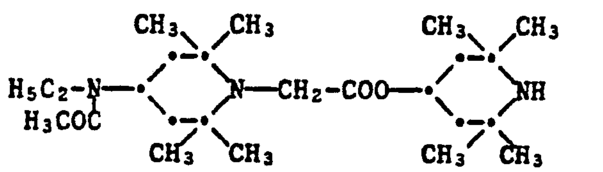

und

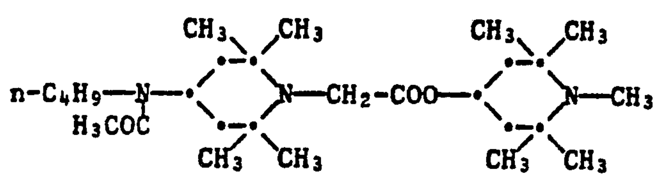

6. Organisches Material enthaltend eine oder mehrere Verbindungen der Formel (I) gemäss Anspruch 1.

7. Polyfine enthaltend eine oder mehrere verbindungen der Formel (I) gemäss Anspruch 1.

**Claims**

1. A compound of the general formula (I)

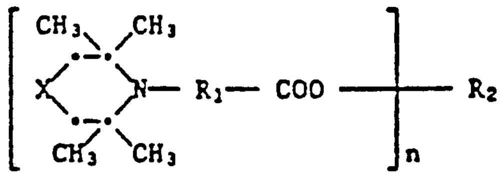

(I)

in which $R_1$ is $C_1$-$C_{12}$alkylene, X is methylene, a group $>CH-OR_3$, in which $R_3$ is hydrogen, $C_1$-$C_{18}$alkyl, $C_3$-$C_{12}$alkenyl, $C_7$-$C_{18}$aralkyl $C_1$-$C_{12}$acyl, or X is also

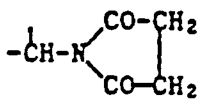 , a group 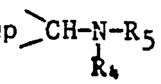 , in which $R_4$ is hydrogen $C_1$-$C_{18}$alkyl which is unsubstituted or substituted by $C_1$-$C_{18}$alkoxy or $C_2$-$C_{18}$dialkylamino, or $R_4$ is also $C_5$-$C_{18}$cycloalkyl, $C_3$-$C_{18}$alkenyl or $C_7$-$C_{18}$aralkyl and $R_5$ is $C_1$-$C_{12}$acyl or a group $-COOR_6$ in which $R_6$ is $C_1$-$C_{12}$alkyl or $C_3$-$C_{12}$alkenyl, or X is a group

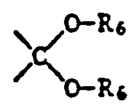

in which $R_6$ is as defined above or X is a group of the formula (II)

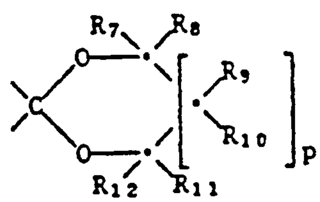

(II),

in which the radicals $R_7$ to $R_{12}$ independently of one another are hydrogen or $C_1$-$C_4$alkyl and p is 0 or 1, n is 1 or 2 and, if n = 1, $R_2$ is a group of the formula (III) or (IV)

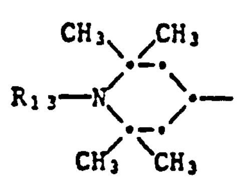

(III)

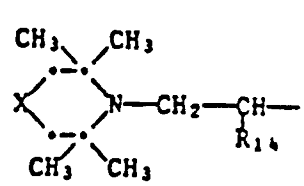

(IV)

in which $R_{13}$ is a hydrogen O·, cyanomethyl, $C_1$-$C_{12}$alkyl, $C_3$-$C_{12}$alkenyl or $C_3$-$C_{12}$alkynyl, $C_7$-$C_{12}$aralkyl or $C_1$-$C_{12}$acyl, X is as defined above and $R_{14}$ is hydrogen, $C_1$-$C_6$alkyl or $C_6$-$C_{12}$aryl, or, if n = 2, $R_2$ is a group of the formula (V)

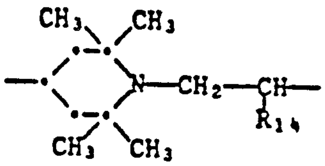 (V)

in which $R_{14}$ is as defined above.

2. A compound of the formula (I) according to claim 1, in which $R_1$ is $C_1$-$C_6$alkylene, X is methylene, a group $>CH-OR_3$ in which $R_3$ is hydrogen, $C_1$-$C_{12}$alkyl, $C_3$-$C_6$alkenyl, $C_7$-$C_9$aralkyl or $C_1$-$C_6$acyl, or X is also a group 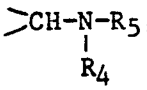, in which $R_4$ is hydrogen, $C_1$-$C_{12}$alkyl which is unsubstituted or substituted by $C_1$-$C_{12}$alkoxy or $C_2$-$C_{12}$dialkylamino, or $R_4$ is $C_6$-$C_9$cycloalkyl, $C_3$-$C_6$alkenyl or $C_7$-$C_9$aralkyl and $R_5$ is $C_1$-$C_6$acyl or a group $-COOR_6$ in which $R_6$ is $C_1$-$C_4$alkyl or X is a group of the formula (II) in which $R_7$ to $R_{12}$ are hydrogen, methyl or ethyl, n is 1 or 2 and, if n = 1, $R_2$ is a group of the formula (III) or (IV) in which $R_{13}$ is hydrogen, $C_1$-$C_6$alkyl, $C_3$-$C_6$alkenyl or $C_3$-$C_6$alkynyl, benzyl or $C_1$-$C_6$acyl, X is defined as above and $R_{14}$ is hydrogen, $C_1$-$C_4$alkyl or phenyl, and if n = 2, $R_2$ is a group of the formula (V) in which $R_{14}$ is as defined above.

3. A compound of the formula (I) according to claim 1, in which $R_1$ is methylene, X is methylene, 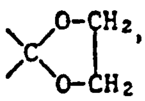, a group $>CH-OR_3$ in which $R_3$ is $C_1$-$C_8$alkyl or allyl, or X is also a group, 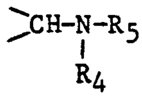 in which $R_4$ is $C_1$-$C_4$alkyl and $R_5$ is $C_2$-$C_4$acyl n is 1 or 2 and if n = 1, $R_2$ is a group of the formula (III) or (IV) in which $R_{13}$ is hydrogen or methyl, X is as defined above and $R_{14}$ is hydrogen or methyl, and, if n = 2, $R_2$ is a group of the formula (V) in which $R_{14}$ is as defined above.

4. A compound of the formula (I) according to claim 1, in which $R_1$ is methylene or propylene, X is methylene, 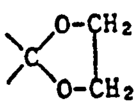, a group, $>CHOR_3$ in which $R_3$ is n-propyl, n-butyl, n-octyl or allyl, or X is a group 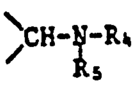 in which $R_4$ is ethyl or n-butyl and $R_5$ is acetyl or $-COOC_2H_5$, n is 1 or 2 and, if n = 1, $R_2$ is a group of the formula (III) or (IV) in which $R_{13}$ is hydrogen or methyl, X is $>CH-OC_4H_9$ and $R_{14}$ is hydrogen, and if n = 2, $R_2$ is a group of the formula (V) in which $R_{14}$ is hydrogen or methyl.

5. Compounds according to claim 1 of the formulae which follow:

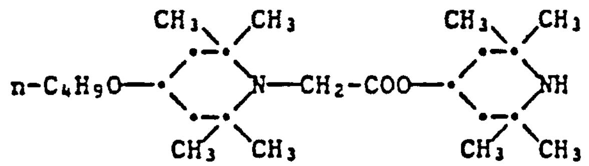

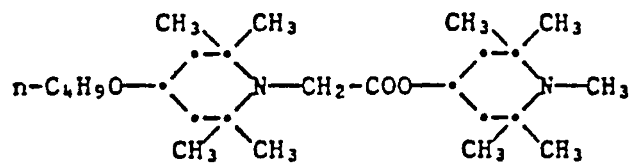

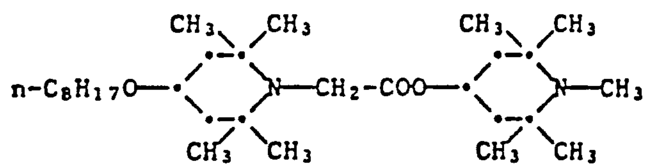

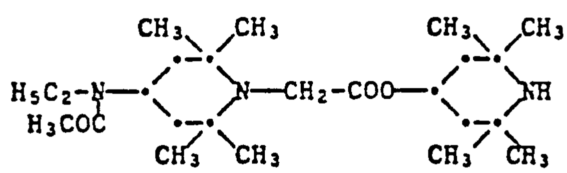

and

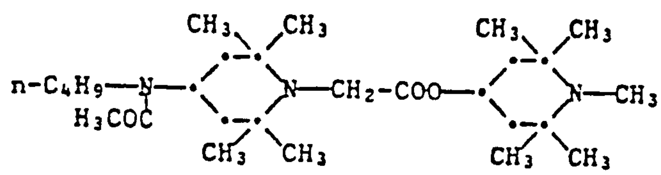

6. Organic material containing one or more compounds of the formula (I) according to claim 1.

7. Polyolefins containing one or more compounds of the formula (I) according to claim 1.

**Revendications**

1. Les composés de formule générale I ci-dessous:

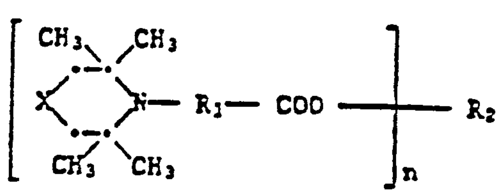

(I),

dans laquelle $R_1$ désigne un alkylène en $C_1$-$C_{12}$, X le groupe méthylène, un groupe $>CH-OR_3$, $R_3$ étant l'hydrogène, un alkyle en $C_1$-$C_{18}$, un alcényle en $C_3$-$C_{12}$, un aralkyle en $C_7$-$C_{18}$ ou un acyle en $C_1$-$C_{12}$, ou encore un groupe

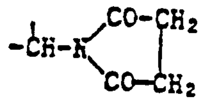 ou 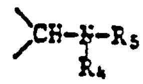 ,

$R_4$ étant l'hydrogène, un alkyle en $C_1$-$C_{18}$ sans substituants ou portant un alcoxy en $C_1$-$C_{18}$ ou un groupe dialkylamino en $C_2$-$C_{18}$, ou encore $R_9$ peut être un cycloalkyle en $C_5$-$C_{18}$, un alcényle en $C_3$-$C_{18}$ ou un aralkyle en $C_7$-$C_{18}$, et $R_5$ étant un acyle en $C_1$-$C_{12}$ ou un groupe -$COOR_6$, $R_6$ étant un alkyle en $C_1$-$C_{12}$ ou un alcényle en $C_3$-$C_{12}$, ou encore X est un groupe

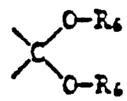

$R_6$ ayant la signification ci-dessus, ou un groupe de formule II:

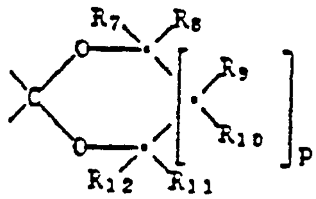 (II),

dans laquelle les radicaux $R_7$ à $R_{12}$ désignent chacun, indépendamment les une des autres, l'hydrogène ou un alkyle en $C_1$-$C_4$, et p est le nombre 0 ou 1, n est le nombre 1 ou 2 et, si n = 1, $R_2$ est un groupe de formule III ou IV ci-dessous:

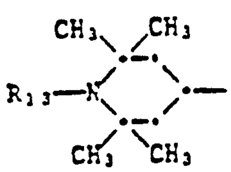
(III)

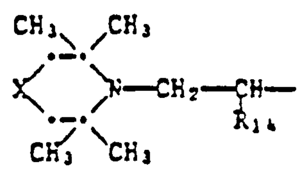
(IV)

$R_{13}$ représentant l'hydrogène, 0·, un groupe cyanométhyle, alkyle en $C_1$-$C_{12}$, alcényle en $C_3$-$C_{12}$, alcynyle en $C_3$-$C_{12}$, aralkyle en $C_7$-$C_{12}$ ou acyle en $C_1$-$C_{12}$, X ayant la signification précédemment donnée, et $R_{14}$ représentant l'hydrogène, un alkyle en $C_1$-$C_6$ ou un aryle en $C_6$-$C_{12}$, alors que si n = 2, $R_2$ est un groupe de formule V:

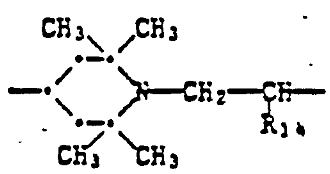 (V),

$R_{14}$ ayant la signification précédemment donnée.

2. Un composé de formule I selon la revendication 1 dans lequel $R_1$ est un alkylène en $C_1$-$C_6$, X le groupe méthylène, un groupe

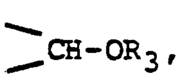 , $R_3$ désignant l'hydrogène, un alkyle en $C_1$-$C_{12}$, un alcényle en $C_3$-$C_6$, un aralkyle en $C_7$-$C_9$, ou un acyle en $C_1$-$C_6$, ou encore un groupe 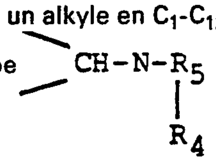 ,

$R_4$ étant l'hydrogène, un alkyle en $C_1$-$C_{12}$ sans substituants ou portant un alcoxy en $C_1$-$C_{12}$ ou un groupe dialkylamino en $C_2$-$C_{12}$, ou encore $R_4$ peut être un cyclo-alkyle en $C_6$-$C_9$, un alcényle en $C_3$-$C_6$ ou un aralkyle en $C_7$-$C_9$, et $R_5$ étant un acyle en $C_1$-$C_6$ ou un groupe -$COOR_6$, $R_6$ étant un alkyle en $C_1$-$C_9$, ou encore X est un groupe de formule II ci-dessus dans lequel les symboles $R_7$ à $R_{12}$ désignent chacun l'hydrogène ou le groupe

méthyle ou éthyle, n = 1 ou 2 et si n = 1, $R_2$ est un groupe de formule III ou IV ci-dessus dans lequel $R_3$ désigne l'hydrogène, un alkyle en $C_1$-$C_6$, un alcényle en $C_3$-$C_6$, un alcynyle en $C_3$-$C_6$, un benzyle ou un acyle en $C_1$-$C_6$, X a la signification précédemment donnée, et $R_{14}$ est l'hydrogène, un alkyle en $C_1$-$C_4$ ou un phényle, alors que si n = 2, $R_2$ est un groupe de formule V ci-dessus dans lequel $R_4$ a la signification déjà donnée.

3. Un composé de formule I selon la revendication 1 dans lequel $R_1$ est le groupe méthylène, X le groupe méthylène ou un groupe:

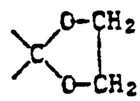 ou 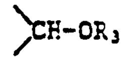,

$R_3$ étant un alkyle en $C_1$-$C_8$ ou le groupe allyle, ou encore

X peut être un groupe 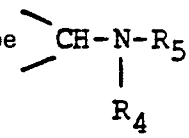 dans lequel $R_4$ est un alkyle en $C_1$-$C_4$ et $R_5$ un acyle en $C_2$-$C_4$, n est le nombre 1 ou 2 et si n = 1, $R_2$ est un groupe de formule III ou IV, $R_{13}$ désignant l'hydrogène ou le groupe méthyle, X ayant la signification précédente et $R_9$ étant l'hydrogène ou un méthyle, tandis que si n = 2 $R_2$ est un groupe de formule V dans lequel $R_{14}$ a la signification précédente.

4. Un composé de formule I selon la revendication 1 dans lequel $R_1$ est le groupe méthylène ou propylène, X le groupe méthylène ou un groupe:

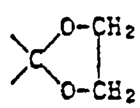 ou 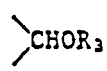,

$R_3$ étant le groupe n-propyle, n-butyle, n-octyle ou allyle, ou encore X est un groupe

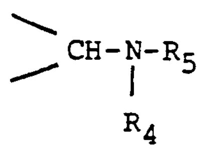

dans lequel $R_4$ est le groupe éthyle ou n-butyle et $R_5$ le groupe acétyle ou $-COOC_2H_5$, n est le nombre 1 ou 2 et si n = 1 $R_2$ est un groupe de formule III ou IV ci-dessus, $R_{13}$ désignant l'hydrogène ou un méthyle, X un groupe

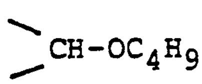

et $R_{14}$ l'hydrogène, tandis que si n = 2 $R_2$ est un groupe de formule V dans lequel $R_{14}$ est l'hydrogène ou le groupe méthyle.

5. Les composés conformes à la revendication 1 de formules suivantes:

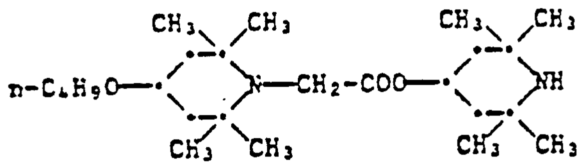

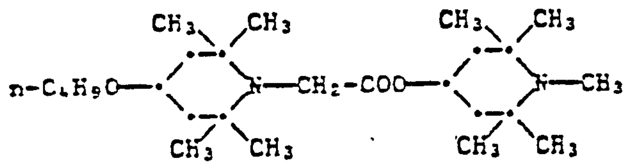

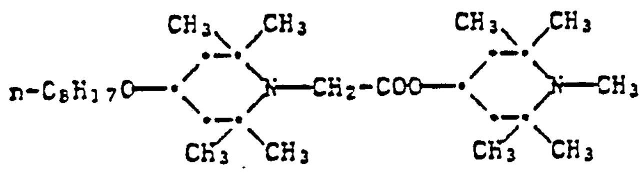

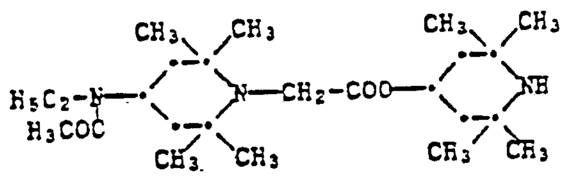

et

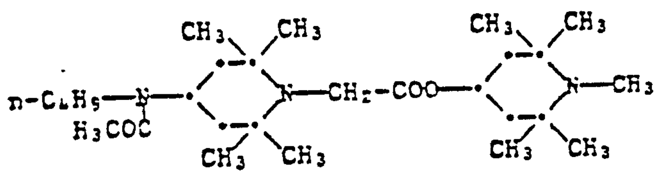

.

6. Matière organique contenant un ou plusieurs composés de formule I selon la revendication 1.

7. Polyoléfines contenant un ou plusieurs composés de formule I selon la revendication 1.